# EUROPÄISCHE PATENTSCHRIFT

(11) **EP 1 743 935 B1**
(45) Veröffentlichungstag und Bekanntmachung des Hinweises auf die Patenterteilung: **11.06.2008**
(21) Anmeldenummer: 06013213.1
(22) Anmeldetag: 27.06.2006
(51) Int. Cl.: C12M 1/12, C12M 1/26

(54) **Gerät zum Sterilisieren und Abfüllen von sterilen flüssigen Medien**
Device to be sterilised and for filling with fluid media
Dispositif pour stériliser et pour le remplissage avec des medias fluides

(30) Priorität: 13.07.2005 DE 202005011194 U
(43) Veröffentlichungstag der Anmeldung: 17.01.2007
(73) Patentinhaber: Systec GmbH Labor-Systemtechnik, 35435 Wettenberg (DE)
(72) Erfinder: Tegeler, Dieter, 49504 Lotte (DE)
(74) Vertreter: Linnemann, Winfried

(56) Entgegenhaltungen:
- EP-A1- 0 741 087
- EP-A2- 0 295 408
- EP-A2- 0 350 723
- EP-A2- 0 357 998
- DE-A1- 3 539 798
- DE-C1- 3 816 935

## Beschreibung

Die Erfindung betrifft einen Abfüllstutzen eines Gerätes zum Sterilisieren und Abfüllen von sterilen flüssigen Medien, insbesondere von mikrobiologischen Medien, wobei der Abfüllstutzen einen unteren Anschlussstutzen und einen oberen Anschlussstutzen aufweist und einen Deckel des Gerätes durchdringend mit dem Deckel verbindbar ist, wobei in einem mit dem Deckel verbundenen Zustand des Abfüllstutzens der untere Anschlussstutzen unterhalb des Deckels und der obere Anschlussstutzen oberhalb des Deckels liegt.

Geräte oder Vorrichtungen zum Sterilisieren und Abfüllen von sterilen flüssigen Medien sind als sogenannte Medienpräparatoren oder Autoklaven bekannt. Ein aus dem praktischen Einsatz bekanntes Gerät der Anmelderin umfasst einen Behälter mit einem Deckel, durch den etwa senkrecht ein Abfüllstutzen verläuft. Am seinem unteren, im Behälter befindlichen Ende ist der Abfüllstutzen über einen unteren Anschlussstutzen mit einem flexiblen Schlauch verbunden, der bis zum tiefsten Punkt des Behälters reicht. Oberhalb des Deckels, und somit außerhalb des Behälters, ist der Abfüllstutzen über einen oberen Anschlussstutzen mit einem Abfüllschlauch verbindbar, über den das im Behälter befindliche Medium, z. B. eine sterile Nährlösung für Pflanzen oder Bakterien, aus dem Behälter abgezogen werden kann, um danach beispielsweise in Nährschalen oder andere Behälter abgefüllt werden zu kön-nen.

Bei dem vorgenannten Gerät muss sichergestellt sein, dass das Medium und alle mit dem Medium in Berührung kommenden Teile zuverlässig sterilisiert sind. Dies ist für das Innere des Behälters einfach und sicher durchzuführen. Das Sterilisieren des Abfüllstutzens ist jedoch bisher deshalb problematisch, weil er zum Teil außerhalb des Behälters liegt und damit in einem Bereich, der beim Erhitzen des Inneren des Behälters nicht mit erhitzt wird und folglich auch nicht steril wird.

Die Europäische Patentschrift EP 0 295 408 B1 zeigt einen sogenannten Bioreaktor, der ein abgedichtetes Gefäß und ein durch die Außenwand des Gefäßes hindurchgehendes Ventil mit einem Ventilgehäuse umfasst, das einen in einer Bohrung verschiebbaren Schieber mit einem in Längsrichtung durchgehenden Kanal enthält. Dabei ist am Ventilgehäuse im Kopfraum des Gefäßes eine durch den Schieber verschließbare Öffnung vorgesehen und das Ventilgehäuse weist unterhalb der Öffnung einen Sitz für den Schieber auf. Hier wird vorgeschlagen, am unteren Ende der Bohrung eine Erweiterung vorzusehen, durch die bei angehobenem Schieber der untere Teil der Bohrung mit dem Kanal in Verbindung steht. Diese Anordnung führt insbesondere infolge der beweglichen Schieber zu einer komplizierten Fertigung und Montage sowie Sterilisierung des Geräts insgesamt.

Die Deutsche Offenlegungsschrift DE 35 39 798 A1 zeigt ein gattungsfremdes Gerät, nämlich eine Vorrichtung zum Begasen von Flüssigkeiten, insbesondere von Kulturmedien zur Vermehrung von Gewebekulturen mit einem Gasaustausch über von einem Träger getragene und auf Halterungen aufgewickelte Membranschläuche. Letztere werden in einem die Flüssigkeit enthaltenden Behälter bewegt, wobei sie über flexible Verbindungsschläuche mit Anschlussstutzen im Deckel des Behälters verbunden sind. Dabei ist weiterhin vorgesehen, dass der durch den Deckel geführte Anschlussstutzen für den Verbindungsschlauch durch einen Schieber ersetzt ist, der in der einen Schieberstellung den angeschlossenen Verbindungsschlauch und damit die Membranschläuche mit dem Innendruck des Behälters und in der anderen Schieberstellung mit dem Begasungsmedium beaufschlagt. Gemäß Figur 2 dieser Druckschrift hat der Anschlussstutzen in seinem im Inneren des Behälters liegenden Teil radiale Bohrungen, die hier jedoch nicht dazu dienen, heißen Dampf zum Zwecke einer Sterilisation nach oben durch den außerhalb des Behälters liegenden Teil des Abfüllstutzens zu leiten.

Ausgehend von den oben geschilderten Gegebenheiten liegt der Erfindung das Bestreben zu Grunde, den Abfüllstutzen eines Gerätes zum Sterilisieren und Abfüllen von sterilen flüssigen Medien so auszubilden und anzuordnen, dass auch der Abfüllstutzen des Gerätes einfach und sicher sterilisiert werden kann.

Um dies zu erreichen, schlägt die Erfindung vor, dass der Abfüllstutzen zwei Teile umfasst, nämlich
a) eine äußere rohrförmige Hülse, die nahe ihren beiden Enden jeweils zumindest eine untere radiale Durchgangsöffnung und obere radiale Durchgangsöffnung und an ihrem unteren Ende den unteren Anschlussstutzen aufweist, und
b) eine innere rohrförmige Hülse, die den oberen Anschlussstutzen aufweist, wobei die innere Hülse in ihrem mit der äußeren Hülse zusammengebauten Zustand gegen die äußere Hülse abgedichtet ist, nur mit dem unteren Anschlussstutzen der äußeren Hülse in Verbindung steht und an oder nahe ihrem oberen Ende durch lösbare Haltemittel axial an der äußeren Hülse gehalten ist, und
dass entweder die obere Durchgangsöffnung als Drosselborung ausgebildet ist oder an die obere Durchgangsöffnung eine Drosselleitung anschließbar ist, wobei die Drosselbohrung oder die Drosselleitung eine Drosselstelle für im Gerät zum Sterilisieren erzeugten, die äußere Hülse in ihrem mit dem Deckel verbundenen Zustand durchströmenden Heißdampf bildet.

Diese Ausbildung und Anordnung des Abfüllstutzens hat zur Folge, dass sowohl das Innere des Behälters als auch der Abfüllstutzen in einfacher und sicherer Weise sterilisiert werden können. So kann die äußere Hülse des Abfüllstutzens in einem mit dem Deckel des Gerätes verbundenen Zustand zusammen mit dem Inneren des Behälters, einschließlich des Mediums, mit Hilfe von hindurchgeleitetem Heißdampf sterilisiert werden. Bei der Sterilisierung strömt heißer Dampf, der im Inneren des Behälters erzeugt wird, durch die unteren, radialen Durchgangsöffnungen in das Innere der äußeren Hülse ein, strömt durch diese hindurch nach oben und verlässt sie durch die obere Durchgangsöffnung. Auf diese Weise wird die äußere Hülse des Abfüllstutzens gleichzeitig mit dem gesamten Behälter zuverlässig sterilisiert. Die Größe der oberen Durchgangsöffnung ist so zu bemessen, dass möglichst nur eine relativ geringe Menge an heißem Dampf hindurchtreten kann; sie sollte somit für den Dampf eine Drosselbohrung darstellen. Um die vorgenannte Drosselwirkung zu erzielen, kann alternativ vorgesehen werden, dass an die Durchgangsöffnung nahe dem oberen Ende der äußeren Hülse eine Drosselleitung anschließbar ist. Diese Drosselleitung kann z. B. die Form eines insbesondere flexiblen Schlauches mit relativ kleinem Durchmesser und relativ großer Länge haben. Der Schlauch kann außen an der oberen Durchgangsöffnung angesetzt werden, die dann auch größer ausgebildet sein kann, da sie in diesem Fall von der Drosselfunktion befreit ist. Hierdurch wird erreicht, dass im Inneren des Behälters ein überdruck gegenüber der Umgebung erzeugbar ist, der für eine deutliche Erhöhung des Siedepunktes für die Sterilisation sorgt, für die z. B. eine Temperatur von etwa 115°C nötig ist.

Die innere Hülse des Abfüllstutzens kann hingegen zunächst als Einzelteil separat in beliebiger Weise sterilisiert und separat verpackt zusammen mit dem Behälter ausgeliefert werden. Erst der Anwender setzt zur Komplettierung des Abfüllstutzens die innere Hülse in die äußere Hülse mit wenigen Handgriffen ein. Somit ist eine sterile Ausbildung des gesamten Gerätes sichergestellt. Bewegliche Schieber oder dergleichen technisch aufwendige Teile sind dabei vermieden, so dass Fertigung und Montage vereinfacht sind.

Die Abdichtung zwischen den beiden konzentrisch angeordneten Hülsen des Anschlussstutzens kann in unterschiedlicher Weise durchgeführt werden. So kann zur Abdichtung zwischen der inneren und der äußeren Hülse ein Dichtring zwischen dem unteren Ende der inneren Hülse und dem unteren Anschlussstutzen der äußeren Hülse angeordnet sein.

Zur Erzielung einer guten, sicheren Abdichtung kann vorgesehen sein, dass die äußere Hülse nahe dem unteren Anschlussstutzen einen Ringbund aufweist, der aus einer Verkleinerung ihres Innendurchmessers auf den Innendurchmesser des unteren Anschlussstutzens gebildet ist, und dass der Dichtring sich einerseits an dem Ringbund der äußeren Hülse und andererseits am unteren Ende der inneren Hülse abstützt.

Eine Ausführung der Abdichtung kann darin bestehen, dass der Dichtring als O-Ring ausgebildet ist, der sich einerseits an dem Ringbund der äußeren Hülse und andererseits an der unteren Stirnfläche der inneren Hülse abstützt, und dass die radiale Durchgangsöffnung bzw. die unteren radialen Durchgangsöffnungen oberhalb des O-Rings angeordnet ist/sind.

Eine bevorzugte Ausführung der Abdichtung sieht vor, dass der Dichtring als Flachring ausgebildet ist, der sich einerseits an dem Ringbund der äußeren Hülse und andererseits an der unteren Stirnfläche der inneren Hülse abstützt und gleichzeitig die untere radiale Durchgangsöffnung bzw. Durchgangsöffnungen verschließt. Durch die vorgenannte Abdichtung zumindest der unteren radialen Durch-gangsöffnungen wird vermieden, dass bei der Entnahme des flüssigen Mediums Nebenluft aus dem Behälter angesaugt wird.

Zur Vormontage der Dichtung wird vorgesehen, dass der Dichtring auf einem im Durchmesser verjüngten unteren Endabschnitt der inneren Hülse sitzt.

Zum schnellen Vervollständigen des Abfüllstutzens, aber auch zur Sicherstellung des Anpressdrucks an der Dichtung, wird vorgesehen, dass als lösbares Haltemittel eine Überwurfmutter dient, die mit einer im Durchmesser verkleinerten axialen Ringfläche auf einen verdickten Bund der inneren Hülse einwirkt, indem sie mit ihrem Innengewinde auf ein Außengewinde der äußeren Hülse aufschraubbar ist. Die Überwurfmutter hält bzw. drückt somit die beiden Hülsen axial zusammen und verpresst gleichzeitig dazwischen den vorgenannten Dichtring.

Um einen störungsfreien, strömungsgünstigen Durchfluss des zu entnehmenden sterilen flüssigen Mediums durch den Abfüllstutzen zu erzielen, wird insbesondere vorgesehen, dass der Innendurchmesser des unteren Anschlussstutzens dem Innendurchmesser der inneren Hülse einschließlich dessen oberen Anschlussstutzens entspricht.

Zur Befestigung des Abfüllstutzens an dem Deckel des Behälters bietet es sich an, dass die äußere Hülse insbesondere nahe ihrem unteren Ende einen geeigneten Stutzen umfasst.

Während der Sterilisierung des Behälters einschließlich der äußeren Hülse wird vorgesehen, dass die äußere Hülse vor dem Einsetzen der inneren Hülse von einer Schraubkappe dicht verschlossen ist. Diese kann auf das für die genannte Überwurfmutter verwendete Außengewinde aufgeschraubt werden.

Damit alle Bauteile des Abfüllstutzens den auftretenden Temperaturen gewachsen sind, wird vorgeschlagen, dass die beiden Hülsen und die eventuelle Schraubkappe aus Metall oder aus einem gegen Heißdampf beständigen Kunststoff bestehen.

Desgleichen sollen auch die Dichtringe aus einem gegen den verwendeten Heißdampf beständigen Kunststoff bestehen. Hierzu kann vorzugsweise ein Fluorkunststoff, wie z. B. Polytetrafluorethylen, dienen, das unter der Marke TEFLON im Handel ist.

Weitere besondere Vorteile kann der Fachmann auch der nachfolgenden Beschreibung eines Ausführungsbeispiels anhand einer Zeichnung entnehmen. Die Figuren der Zeichnung zeigen:
- Figur 1: die äußere Hülse eines Abfüllstutzens mit abgedichteter Schraubkappe, in Seitenansicht,
- Figur 2: die Hülse nach Figur 1 im Längsschnitt nach der Linie I-I in Figur 1,
- Figur 3: einen Abfüllstutzen, bei dem die äußere Hülse nach Figur 1 mit einer inneren Hülse komplettiert ist, in Seitenansicht, und
- Figur 4: den Abfüllstutzen nach Figur 3 im Längsschnitt nach der Linie IV - IV in Figur 3.

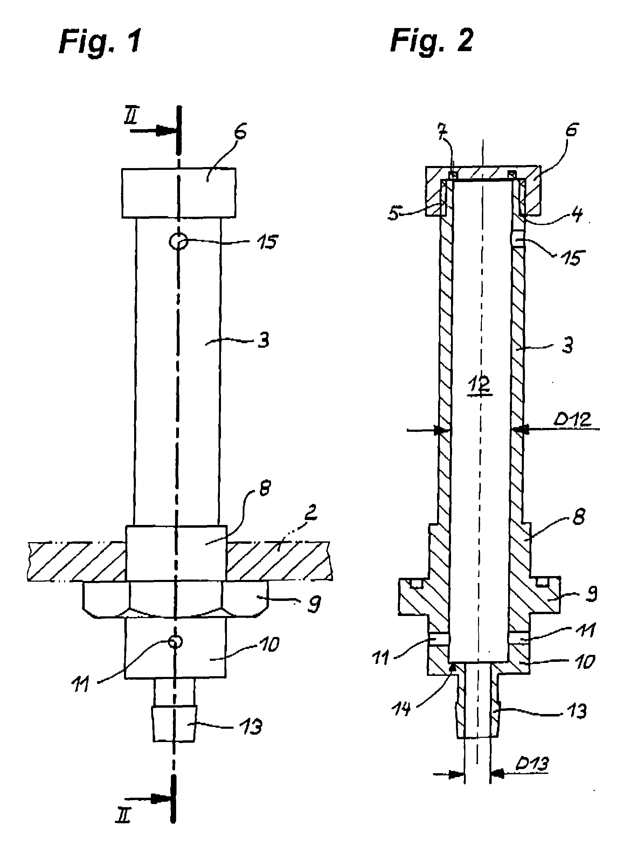

In den Figuren 1 und 2 ist ein bei einem Gerät zum Sterillisieren und Abfüllen von sterilen flüssigen Medien verwendeter Abfüllstutzen 1 während des Sterilisierens dargestellt. In diesem Zustand durchdringt der Abfüllstutzen 1 einen im Schnitt angedeuteten Deckel 2 eines Behälters des Gerätes, das im Übrigen nicht dargestellt ist. Der Abfüllstutzen 1 besteht zu diesem Zeitpunkt zunächst nur aus einer äußeren rohrförmigen Hülse 3, die an ihrem oberen Ende ein Außengewinde 4 aufweist und mit einer mit einem entsprechenden Innengewinde 5 versehene Schraubkappe 6 und einem Dichtring 7 verschlossen ist.

Die Hülse 3 durchdringt mit einem Abschnitt 8 den Deckel 2 und ist in nicht näher dargestellter Weise mit Hilfe eines Stutzens 9 dicht in dem Deckel 2 befestigt. Unterhalb des Stutzens 9 ist der Hülse 3 ein Abschnitt 10 angeformt, aus dem zwei oder mehr radiale Durchgangsöffnungen 11 austreten, die mit der inneren axialen Bohrung 12 der Hülse 3 in Verbindung stehen. An den Abschnitt 10 schließt sich nach unten ein unterer Anschlussstutzen 13 an. Der Abschnitt 10 verjüngt sich hierzu vom inneren Durchmesser D12 zu einem kleineren inneren Durchmesser D13 und bildet dabei einen Ringbund 14.

Nahe dem oberen Ende der Hülse 3 ist eine radiale Durchgangsöffnung 15 in Form einer kleinen Drosselbohrung angebracht, welche die axiale Bohrung 12 mit der Umgebung verbindet.

In diesem Zustand kann im Inneren des Behälters, also unterhalb des Deckels 2, zum Zweck einer Sterilisation erzeugter Heißdampf unter erhöhtem Druck durch die radialen Durchgangsöffnungen 11 in die Bohrung 12 eintreten und durch die Drosselbohrung 15 wieder in die Umgebung austreten, so dass eine gute Sterilisation des Inneren der Hülse 3 erzielt wird.

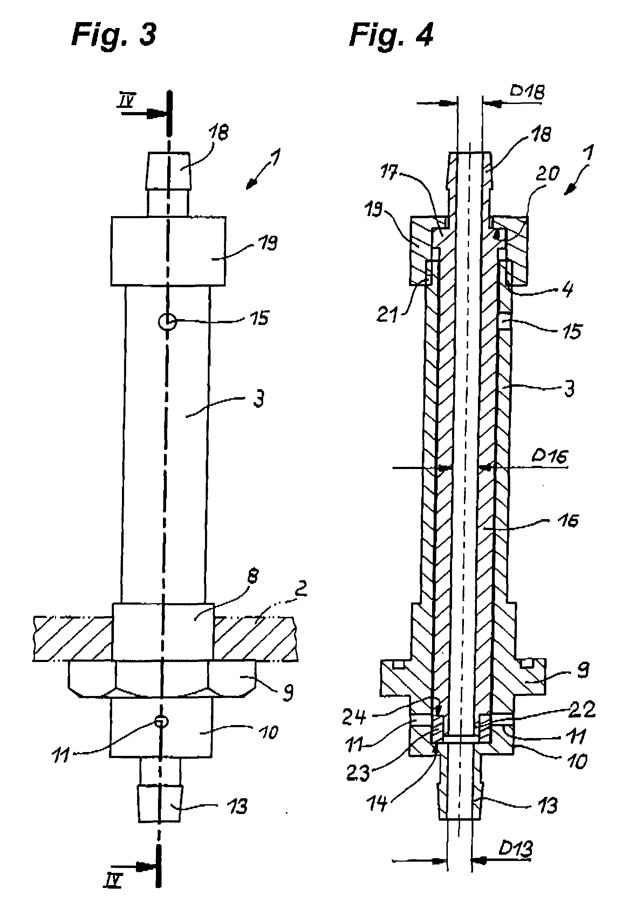

In den Figuren 3 und 4 ist der Abfüllstutzen 1 in zusammengebautem, komplettiertem Zustand dargestellt, wie er zum Abfüllen gebraucht wird. Die Schraubkappe 6 ist entfernt und stattdessen ist in die axiale Bohrung 12 eine innere rohrförmige Hülse 16 eingeschoben. Der Hülse 16 ist oben ein im Durchmesser leicht verdickter Bund 17 und daran endseitig ein oberer Anschlussstutzen 18 angeformt. Der Bund 17 und der Anschlussstutzen 18 ragen oben aus der äußeren Hülse 3 heraus. Die innere Hülse 16 wird an der äußeren Hülse 3 durch eine Überwurfmutter 19 gehalten, die axial über den Anschlussstutzen 18 geschoben ist, mit einer Ringfläche 20 an dem Bund 17 axial anliegt und mittels eines Innengewindes 21 auf das Außengewinde 4 aufschraubbar ist.

Am unteren Ende der Hülse 16 ist ein im Außendurchmesser verminderter axialer Abschnitt 22 angeformt, auf den ein breiter Flachdichtring 23 aufgeschoben ist, der sich einerseits an dem Ringbund 14 der Hülse 3 und andererseits an der oberen Stirnfläche 24 des Abschnitts 22 axial abstützt, wobei diese Abstützung durch das genannte Aufschrauben der Überwurfmutter 19 bewirkt wird. Gleichzeitig verschließt der Flachdichtring 23 die unteren radialen Durchgangsöffnungen 11.

Aus Figur 4 wird deutlich, dass der innere Durchmesser des komplettierten Abfüllstutzens 1 über seine gesamte axiale Länge praktisch gleichbleibend groß ist; die Durchmesser D13, D16 und D18 sind nämlich gleich groß.

Die innere Hülse 16 kann schon werkseitig sterilisiert angeliefert und bei Bedarf in die zusammen mit dem Behälter durch Heißdampf sterilisierte äußere Hülse 3 eingebaut werden, so dass eine gute Sterilisation des gesamten Abfüllstutzens 1 sichergestellt ist.

### Bezugszeichenliste:

| Zeichen | Bezeichnung |
|---|---|
| 1 | Abfüllstutzen |
| 2 | Deckel |
| 3 | äußere Hülse |
| 4 | Außengewinde |
| 5 | Innengewinde |
| 6 | Schraubkappe |
| 7 | Dichtring |
| 8 | Abschnitt |
| 9 | Stutzen |
| 10 | Abschnitt |
| 11 | Durchgangsöffnung |
| 12 | Bohrung |
| 13 | Anschlussstutzen |
| 14 | Ringbund |
| 15 | Durchgangsöffnung |
| 16 | innere Hülse |
| 17 | Bund |
| 18 | Anschlussstutzen |
| 19 | Überwurfmutter |
| 20 | Ringfläche |
| 21 | Innengewinde |
| 22 | Abschnitt |
| 23 | Flachdichtring |
| 24 | Stirnfläche |
| D12 | innerer Durchmesser von 12 |
| D13 | innerer Durchmesser von 13 |
| D16 | innerer Durchmesser von 16 |
| D18 | innerer Durchmesser von 18 |

## Patentansprüche

1. Abfüllstutzen (1) eines Gerätes zum Sterilisieren und Abfüllen von sterilen flüssigen Medien, insbesondere von mikrobiologischen Medien, wobei der Abfüllstutzen (1) einen unteren Anschlussstutzen (13) und einen oberen Anschlussstutzen (18) aufweist und einen Deckel (2) des Gerätes durchdringend mit dem Deckel (2) verbindbar ist, wobei in einem mit dem Deckel (2) verbundenen zustand des Abfüllstutzens (1) der untere Anschlussstutzen (13) unterhalb des Deckels (2) und der obere Anschlussstutzen (18) oberhalb des Deckels (2) liegt,
**dadurch gekennzeichnet,**
**dass** der Abfüllstutzen (1) zwei Teile umfasst, nämlich
a) eine äußere rohrförmige Hülse (3), die nahe ihren beiden Enden jeweils zumindest eine untere radiale Durchgangsöffnung (11) und obere radiale Durchgangsöffnung (15) und an ihrem unteren Ende den unteren Anschlussstutzen (13) aufweist, und
b) eine innere rohrförmige Hülse (16), die den oberen Anschlussstutzen (18) aufweist, wobei die innere Hülse (16) in ihrem mit der äußeren Hülse (3) zusammengebauten zustand gegen die äußere Hülse (3) abgedichtet ist, nur mit dem unteren Anschlussstutzen (13) der äußeren Hülse (3) in Verbindung steht und an oder nahe ihrem oberen Ende durch lösbare Haltemittel (19) axial an der äußeren Hülse (3) gehalten ist, und
**dass** entweder die obere Durchgangsöffnung (15) als Drosselbohrung ausgebildet ist oder an die obere Durchgangsöffnung (15) eine Drosselleitung anschließbar ist, wobei die Drosselbohrung oder die Drosselleitung eine Drosselstelle für im Gerät zum Sterilisieren erzeugten, die äußere Hülse (3) in ihrem mit dem Deckel (2) verbundenen Zustand durchströmenden Heißdampf bildet.

2. Abfüllstutzen nach Anspruch 1, **dadurch gekennzeichnet, dass** zur Abdichtung zwischen der inneren Hülse (16) und der äußeren Hülse (3) ein Dichtring (23) zwischen dem unteren Ende (22) der inneren Hülse (16) und dem unteren Anschlussstutzen (13) der äußeren Hülse (3) angeordnet ist.

3. Abfüllstutzen nach Anspruch 2, **dadurch gekennzeichnet, dass** die äußere Hülse (3) nahe dem unteren Anschlussstutzen (13) einen Ringbund (14) aufweist, der aus einer Verkleinerung ihres Innendurchmessers (D12) auf den Innendurchmesser (D13) des unteren Anschlussstutzens (13) gebildet ist, und dass der Dichtring (23) sich einerseits an dem Ringbund (14) der äußeren Hülse (3) und andererseits am unteren Ende (22) der inneren Hülse (16) abstützt.

4. Abfüllstutzen nach Anspruch 3, **dadurch gekennzeichnet, dass** der Dichtring (23) als O-Ring ausgebildet ist, der sich einerseits an dem Ringbund (14) der äußeren Hülse (3) und andererseits an einer unteren Stirnfläche (24) der inneren Hülse (16) abstützt und dass die untere radiale Durchgangsöffnung (11) bzw. die unteren radialen Durchgangsöffnungen (11) oberhalb des O-Rings angeordnet ist/sind.

5. Abfüllstutzen nach Anspruch 3, **dadurch gekennzeichnet, dass** der Dichtring (23) als Flachring ausgebildet ist, der sich einerseits an dem Ringbund (14) der äußeren Hülse (3) und andererseits an einer unteren Stirnfläche (24) der inneren Hülse (16) abstützt und gleichzeitig die untere radiale Durchgangsöffnung bzw. Durchgangsöffnungen (11) verschließt.

6. Abfüllstutzen nach einem der Ansprüche 2 bis 5, **dadurch gekennzeichnet, dass** der Dichtring (23) auf einem im Durchmesser verjüngten unteren Endabschnitt (22) der inneren Hülse (16) sitzt.

7. Abfüllstutzen nach einem der Ansprüche 1 bis 6, **dadurch gekennzeichnet, dass** als lösbares Haltemittel eine Überwurfmutter (19) dient, die mit einer im Durchmesser verkleinerten axialen Ringfläche (20) auf einen verdickten Bund (17) der inneren Hülse (16) einwirkt, indem sie mit ihrem Innengewinde (21) auf ein Außengewinde (4) der äußeren Hülse (3) aufschraubbar ist.

8. Abfüllstutzen nach einem der Ansprüche 1 bis 7, **dadurch gekennzeichnet, dass** der Innendurchmesser (D13) des unteren Anschlussstutzens (13) dem Innendurchmesser (D16; D18) der inneren Hülse (16) einschließlich dessen oberem Anschlussstutzens (18) entspricht.

9. Abfüllstutzen nach einem der Ansprüche 1 bis 8, **dadurch gekennzeichnet, dass** die äußere Hülse (3) nahe ihrem unteren Ende einen Stutzen (9) zum Befestigen an dem Deckel (2) des Gerätes aufweist.

10. Abfüllstutzen nach einem der Ansprüche 1 bis 9, **dadurch gekennzeichnet, dass** die äußere Hülse (3) vor dem Einsetzen der inneren Hülse (16) von einer Schraubkappe (6) dicht verschlossen ist.

11. Abfüllstutzen nach einem der Ansprüche 1 bis 10, **dadurch gekennzeichnet, dass** die beiden Hülsen (3, 16) und die eventuelle Schraubkappe (6) aus Metall oder aus einem gegen Heißdampf beständigen Kunststoff bestehen.

12. Abfüllstutzen nach einem der Ansprüche 2 bis 11, **dadurch gekennzeichnet, dass** der Dichtring (23) aus einem gegen Heißdampf beständigen Kunststoff besteht.

## Claims

1. A filling piece (1) of a device for sterilizing and filling sterile liquid media, in particular microbiological media, the filling piece (1) comprising a lower connection piece (13) and an upper connection piece (18), and the filling piece (1) of the device being connectable to the cover (2) in a penetrating manner, wherein, if the filling piece (1) is in a state where it is connected to the cover (2), the lower connection piece (13) is positioned below the cover (2) and the upper connection piece (18) is positioned above the cover (2),
**characterized in that** the filling piece (1) comprises two parts, that are
a) an outer tubular sleeve (3) which, near each of its two ends, comprises at least one lower radial through opening (11) and at least one upper radial through opening (15) and, at its lower end, comprises the lower connection piece (13), and
b) an inner tubular sleeve (16) which comprises the upper connection piece (18), wherein the inner sleeve (16), if in the state where it is assembled to the outer sleeve (3), is sealed against the outer sleeve (3), is only in communication with the lower connection piece (13) of the outer sleeve (3) and, at or near its upper end, is held axially on the outer sleeve (3) by means of detachable holding means (19), and
that either the upper through opening (15) is designed as a throttle hole, or a throttle line can be connected to the upper through opening (15), wherein the throttle hole or the throttle line forms a throttling site for high-temperature steam which is generated in the device for sterilization purposes and flows through the outer sleeve (3) if the latter is in the state where it is connected to the cover (2).

2. The filling piece according to Claim 1, **characterized in that** a sealing ring (23) is arranged between the lower end (22) of the inner sleeve (16) and the lower connection piece (13) of the outer sleeve (3), to provide a seal between the inner sleeve (16) and the outer sleeve (3).

3. The filling piece according to Claim 2, **characterized in that** the outer sleeve (3) comprises a circular flange (14) near the lower connection piece (13), said circular flange (14) being formed through a reduction of its inside diameter (D12) to the inside diameter (D13) of the lower connection piece (13), and that, on the one hand, the sealing ring (23) is supported against the circular flange (14) of the outer sleeve (3) and, on the other hand, against the lower end (22) of the inner sleeve (16).

4. The filling piece according to Claim 3, **characterized in that** the sealing ring (23) is designed as an O-ring which is, on the one hand, supported against the circular flange (14) of the outer sleeve (3) and, on the other hand, against a lower end face (24) of the inner sleeve (16), and that the lower radial through opening (11) or the lower radial through openings (11) is/are arranged above the O-ring.

5. The filling piece according to Claim 3, **characterized in that** the sealing ring (23) is designed as a flat ring which is, on the one hand, supported against the circular flange (14) of the outer sleeve (3) and, on the other hand, against a lower end face (24) of the inner sleeve (16), at the same time closing the lower radial through opening or through openings (11).

6. The filling piece according to anyone of Claims 2 to 5, **characterized in that** the sealing ring (23) is seated on a lower end section (22) of the inner sleeve (16), said lower end section (22) being tapered in diameter.

7. The filling piece according to anyone of Claims 1 to 6, **characterized in that** a coupling nut (19) is provided as detachable holding means, said coupling nut (19) acting on a thickened flange (17) of the inner sleeve (16) with an axial ring surface (20) which is reduced in diameter, this being achieved **in that** said coupling nut (19) can be screwed onto an external thread (4) of the outer sleeve (3) with its internal thread (21).

8. The filling piece according to anyone of Claims 1 to 7, **characterized in that** the inside diameter (D13) of the lower connection piece (13) corresponds to the inside diameter (D16; D18) of the inner sleeve (16) including the upper connection piece (18) thereof.

9. The filling piece according to anyone of Claims 1 to 8, **characterized in that,** near its lower end, the outer sleeve (3) comprises a piece (9) to be mounted to the cover (2) of the device.

10. The filling piece according to anyone of Claims 1 to 9, **characterized in that** the outer sleeve (3) is tightly closed by a screw cap (6) before the inner sleeve (16) is inserted.

11. The filling piece according to anyone of Claims 1 to 10, **characterized in that** the two sleeves (3, 16) and the potential screw cap (6) are made of metal or of a plastic material that is resistant against high-temperature steam.

12. The filling piece according to anyone of Claims 2 to 11, **characterized in that** the sealing ring (23) is made of a plastic material that is resistant against high-temperature steam.

## Revendications

1. Tubulure de remplissage (1) d'un appareil pour la stérilisation et le remplissage de milieux liquides stériles, notamment de milieux microbiologiques, la tubulure de remplissage (1) présentant une tubulure de raccord inférieure (13) et une tubulure de raccord supérieure (18) et pouvant être reliée à un couvercle (2) de l'appareil de manière pénétrante avec le couvercle (2), la tubulure de raccord inférieure (13) se trouvant, dans un état relié au couvercle (2) de la tubulure de remplissage (1), en dessous du couvercle (2) et la tubulure de raccord supérieure (18) au-dessus du couvercle (2),
**caractérisée en ce**
**que** la tubulure de remplissage (1) comprend deux parties, à savoir
a) un manchon tubulaire externe (3) qui présente près de ses deux extrémités à chaque fois au moins une ouverture de passage radiale inférieure (11) et une ouverture de passage radiale supérieure (15) et au niveau de son extrémité inférieure la tubulure de raccord inférieure (13), et
b) un manchon tubulaire interne (16) qui présente la tubulure de raccord supérieure (18), le manchon interne (16) étant étanchéifié dans son état assemblé avec le manchon externe (3) contre le manchon externe (3), n'étant en liaison qu'avec la tubulure de raccord inférieure (13) du manchon externe (3) et étant maintenu de manière axiale au manchon externe (3) au niveau de ou près de son extrémité supérieure par des moyens de maintien amovibles (19), et
**que** l'ouverture de passage supérieure (15) est réalisée en tant qu'orifice d'étranglement ou qu'une conduite d'étranglement peut être raccordée à l'ouverture de passage supérieure (15), l'orifice d'étranglement ou la conduite d'étranglement formant un point d'étranglement pour la vapeur surchauffée générée dans l'appareil pour la stérilisation, s'écoulant à travers le manchon externe (3) dans son état relié au couvercle (2).

2. Tubulure de remplissage selon la revendication 1, **caractérisée en ce qu'un** anneau d'étanchéité (23) est disposé entre l'extrémité inférieure (22) du manchon interne (16) et la tubulure de raccord inférieure (13) du manchon externe (3) pour l'étanchéification entre le manchon interne (16) et le manchon externe (3).

3. Tubulure de remplissage selon la revendication 2, **caractérisée en ce que** le manchon externe (3) présente un collet annulaire (14) près de la tubulure de raccord inférieure (13) qui est formé d'un rétrécissement de son diamètre interne (D12) au diamètre interne (D13) de la tubulure de raccord inférieure (13), et que l'anneau d'étanchéité (23) s'appuie d'un côté sur le collet annulaire (14) du manchon externe (3) et de l'autre sur l'extrémité inférieure (22) du manchon interne (16).

4. Tubulure de remplissage selon la revendication 3, **caractérisée en ce que** l'anneau d'étanchéité (23) est réalisé comme joint torique qui s'appuie d'un côté sur le collet annulaire (14) du manchon externe (3) et de l'autre sur une surface frontale inférieure (24) du manchon interne (16) et que l'ouverture de passage radiale inférieure (11) ou les ouvertures de passage radiales inférieures (11) est/sont disposée(s) au-dessus du joint torique.

5. Tubulure de remplissage selon la revendication 3, **caractérisée en ce que** l'anneau d'étanchéité (23) est réalisé en tant qu'anneau plat qui s'appuie d'un côté sur le collet annulaire (14) du manchon externe (3) et de l'autre sur une surface frontale inférieure (24) du manchon interne (16) et ferme simultanément l'ouverture de passage ou les ouvertures de passage radiale(s) inférieure(s) (11).

6. Tubulure de remplissage selon l'une quelconque des revendications 2 à 5, **caractérisée en ce que** l'anneau d'étanchéité (23) siège sur une portion terminale inférieure (22) au diamètre rétréci du manchon interne (16).

7. Tubulure de remplissage selon l'une quelconque des revendications 1 à 6, **caractérisée en ce qu'un** écrou d'accouplement (19) qui agit avec une surface annulaire axiale (20) au diamètre réduit sur un collet épaissi (17) du manchon interne (16) **en ce qu'**il peut être vissé avec son filetage interne (21) sur un filetage externe (4) du manchon externe (3), sert de moyen de maintien amovible.

8. Tubulure de remplissage selon l'une quelconque des revendications 1 à 7, **caractérisée en ce que** le diamètre interne (D13) de la tubulure de raccord inférieure (13) correspond au diamètre interne (D16 ; D18) du manchon interne (16), y compris de sa tubulure de raccord supérieure (18).

9. Tubulure de remplissage selon l'une quelconque des revendications 1 à 8, **caractérisée en ce que** le manchon externe (3) présente près de son extrémité inférieure une tubulure (9) pour la fixation au couvercle (2) de l'appareil.

10. Tubulure de remplissage selon l'une quelconque des revendications 1 à 9, **caractérisée en ce que** le manchon externe (3) est fermé de manière étanche par un capuchon vissé (6) avant l'insertion du manchon interne (16).

11. Tubulure de remplissage selon l'une quelconque des revendications 1 à 10, **caractérisée en ce que** les deux manchons (3, 16) et le capuchon vissé éventuel (6) se composent de métal ou d'un plastique résistant à la vapeur surchauffée.

12. Tubulure de remplissage selon l'une quelconque des revendications 2 à 11, **caractérisée en ce que** l'anneau d'étanchéité (23) se compose d'un plastique résistant à la vapeur surchauffée.
